(19) Europäisches Patentamt
European Patent Office
Office européen des brevets

(11) Publication number: 0 138 590
B1

(12) # EUROPEAN PATENT SPECIFICATION

(45) Date of publication of patent specification: 06.05.87

(51) Int. Cl.[4]: C 08 G 77/38, C 07 F 7/08, C 07 F 7/18, A 61 K 7/42

(21) Application number: 84306976.6

(22) Date of filing: 12.10.84

(54) **Organosilicon compounds and preparation and use thereof.**

(30) Priority: 15.10.83 GB 8327668

(43) Date of publication of application:
24.04.85 Bulletin 85/17

(45) Publication of the grant of the patent:
06.05.87 Bulletin 87/19

(84) Designated Contracting States:
AT BE CH DE FR GB IT LI NL SE

(56) References cited:
DE-A-1 570 665
FR-A-1 518 350
FR-A-1 527 781
US-A-3 068 152
US-A-3 068 153

(73) Proprietor: Dow Corning Limited
Inveresk House 1 Aldwych
London WC2R 0HF (GB)

(72) Inventor: Hill, Michael Philip Louis
Saint Bleddians Saint Lythans
Near Wenvoe South Glamorgan Wales (GB)

(74) Representative: Walbeoff, William John
Dow Corning Limited Cardiff Road Barry
South Glamorgan CF6 7YL Wales (GB)



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to novel organosilicon compounds which absorb ultra violet radiation and which are useful *inter alia* as sunscreen agents.

A number of organic compounds, generally organic acids and derivatives thereof, are known to have U.S.-absorbing properties and are employed on a commercial scale as ingredients in sunscreen preparations. Although such materials function adequately they are easily removed from the substrate to which they have been applied. For example, cosmetic sunscreen preparations can be removed during bathing thus requiring repeated applications if protection is to be maintained. It is also desirable that the active ingredient remain on the surface of the skin rather than be absorbed thereby.

British Patent 1 164 522 discloses organosilicon compounds which are useful as sunscreen agents and which may be prepared by the rection of an oranosilicon compound having at least one SiH linkage with an organic compound having terminal unsaturation. According to Patent 1 164 522 allyl cinnamate is particularly preferred as the unsaturated organic compound. The use of allyl cinnamate for the preparation of such compounds has, however, been found to be less than satisfactory. Due to the occurrence of secondary rearrangement reactions the yield of the desired product is generally poor.

A method of preparaing organosilicon cinnamates which avoids such secondary reactions is described in British Patent 1 373 458. The said method involves the reaction of allyl cinnamate with a silane or siloxane having silicon-bonded mercaptoalkyl groups. Usually, however, some residual odour of the mercaptoalkyl ractant remains in the product thus rendering it generally unsuitable for cosmetic applications.

FR—A—1 527 781 discloses organosilcon compounds comprising Si-bound p-dialkylamino benzoic acid propyl ester groups and both FR—A—1 518 350 and DE—A—1 570 665 disclose organosilicon compounds comprising Si-bound cinnamic acid propyl ester groups.

According to this invention there are provided organosilicon compounds which are

(1) silanes represented by the general formula

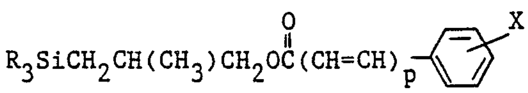

or (2) siloxanes having at least one unit represented by the general formula

(i) 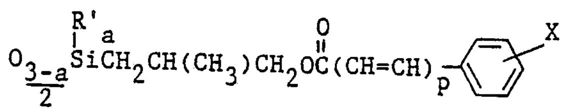

any other units present in the said siloxanes being those represented by the general formula

(ii) $$Z_bSiO_{\frac{4-b}{2}}$$

wherein each R represents a halogen atom, an alkyl group having from 1 to 4 carbon atoms or a phenyl group, an alkoxy group having less than 8 carbon atoms or an alkoxyalkoxy group having less than 8 carbon atoms, R′ represents an alkoxy group having less than 8 carbon atoms, an alkoxyalkoxy group having less than 8 carbon atoms, a methyl group or a phenyl group, X represents a hydroxyl group, methoxy group or ethoxy group when $p = 1$ or a hydroxyl group when $p = 0$, Z represents a hydrogen atom, a monovalent hydrocarbon group or a monovalent halogenated hydrocarbon group, $a$ is 0, 1 or 2, $b$ is 0, 1, 2 or 3 and $p$ is 0 or 1.

In the general formulae of the silanes and siloxanes of this invention each R may be for example chlorine, bromine, methyl, ethyl, methoxy, ethoxy or methoxyethoxy. The R′ substituents may each be methyl, phenyl or an alkoxy or alkoxyalkoxy group having less than 8 carbon atoms e.g. methoxy, ethoxy or methoxyethoxy. Each of the Z substituents may be H, or a monovalent hydrocarbon or halogenated hydrocarbon group, preferably having less than 8 carbon atoms, for example methyl, vinyl, phenyl or 3,3.3-trifluoropropyl.

The siloxanes of this invention have in the molecule at last one unit falling within the general formula (i). They may be homopolymers consisting only of such units (i) or they may be copolymers containing both units (i) and units falling within the general formula (ii). The siloxanes may vary in molecular size from the disiloxanes to high molecular weight homopolymers and copolymers and may range in consistency from freely flowing liquids to gum-like or resinous solids. Preferred, at least for cosmetic applications, are the liquid, substantially linear siloxane homopolymers and copolymers. It is also preferred for such applications that at least 30 percent and preferably at least 70 percent of the R′ and Z substituents are methyl groups.

It has been found that compounds wherein X represents methoxy or ethoxy exhibit high absorbance in the erythemic region (290—320 nm). Such compounds are therefore preferred for use in applications e.g. cosmetic sunscreen products where absorption in this region of the UV spectrum is desired.

The silanes and siloxanes of this invention can be prepared by the reaction of a silane or siloxane having SiH groups with methallylmethoxycinnamate, methallylethoxy cinnamate, methallylhydroxycinnamate or methallyl salicylate. This invention therefore also includes a process for the preparation of organosilicon compounds of the kind specified herein which comprises reacting together (A) a compound of the general formula

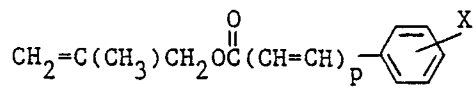

wherein *p* is 0 or 1 and X represents —OH, —$OCH_3$ or —$OC_2H_5$ when *p* is 1, or —OH, when *p* = 0 and (B) an organosilicon compound which is a silane of the general formula

$$R_3SiH$$

or a siloxane having in the molecule at least one unit of the general formula

$$O_{\frac{3-a}{2}}\overset{\overset{\displaystyle R'}{|\ a}}{Si}H$$

any other units present in the siloxane being those represented by the general formula

(ii) $$Z_bSiO_{\frac{4-b}{2}}$$

wherein R', Z, **a** and **b** are as hereinbefore defined.

The reaction between (A) and (B) may be carried out employing known procedures for the addition of silicon-bonded hydrogen atoms to groups containing olefinic unsaturation. Thus, such reactions are generally catalysed by a platinum group metal or a compound or complex of such a metal. Examples of catalysts which may be employed in the reaction between (A) and (B) are platinum on carbon, chloroplatinic acid, platinum acetyl acetonate, complexes of platinum compounds with unsaturated compounds e.g. olefins and vinyl siloxanes, complexes of rhodium and palladium compounds and complexes of platinum compounds supported on inorganic substrates. The addition reaction may be performed at sub-atmospheric, atmospheric or super-atmospheric pressure and in the presence or absence of solvents. It is generally preferred to employ a solvent e.g. toluene or xylene in the reaction mixture. It is also preferred to use elevated reaction temperatures e.g. from about 50°C up to the reflux temperature of the reaction mixture. Preferably stoichiometric proportions of (A) and (B) are employed or a slight stoichiometric excess of (A). However, a stoichiometric deficiency of (A) can be employed if residual silicon-bonded hydrogen is desired in the product.

Siloxanes of the invention can also be prepared from the corresponding hydrolysable silanes by hydrolysis or cohydrolysis or by equilibration of the silanes with cycylic or linear siloxanes.

The silanes and siloxanes of this invention absorb ultra-violet radiation and are therefore useful as agents for preventing sunburn. They may be applied *per se* to the skin but are more preferably formulated into compositions with, for example, inert carriers e.g. solvent such as ethanol, isopropanol, glycerine and mineral oil and cream base materials such as stearic acid, propylene glycol, beeswax and cetyl alcohol. Other conventional ingredients e.g. perfumes and known U.V. absorbing substances may also be included in the formulated compositions. The silanes and siloxanes of the invention are also useful in the coating of substrates, e.g. wood, plastics or metal, to which they may be applied either *per se* or as additives to coating compositions.

The following examples in which Me represents the methyl group illustrate the invention.

Example 1

$Me_3SiO(Me_2SiO)_6(MeHSiO)_2SiMe_3$ (I)
methallyl p-methoxy cinnamate (II)

A platinum catlyst (0.6g) obtained from the reaction of a methylvinylsiloxane oligomer and chloroplatinic acid was dissolved in toluene (7.3g) and the solution charged to a flask fitted with a condenser and dropping funnel. The contents of the flask were then heated to 100°C and a mixture of reactant I (64g), reactant II (46g) and toluene (65g) added slowly from the dropping funnel. After 20 hours a second portion (0.6g) of the platinum catalyst was added and the reaction mixture maintained at about 100°C for a further 6 hours.

Toluene was removed from the reaction mixture by vacuum distillation and the remaining polymer washed twice with aqueous methanol solution. The product was a clear polymer of the average formula

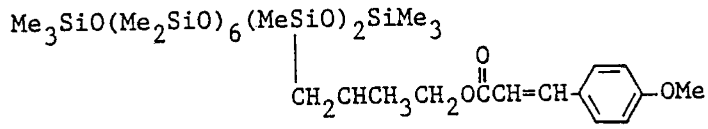

essentially free of II and having a viscosity of 220 × $10^{-6}$ $m^2/s$ at 25°C. The wavelength of maximum absorbtion for the polymer λ max was 308 nm.

The methallyl p-methoxycinnamate employed as reactant in this Example was prepared as follows.

712.8g (4 moles) p-methoxy cinnamic acid were charged to a 6 l flask fitted with stirrer, thermometer, condenser and dropping funnel, and dissolved in 1200ml N,N-dimethyl formamide at 70°C. To this solution 728.6g triethylamine (7.2 moles) and 579.5g (6.4 moles) methayllylchloride were added successively. The reaction mixture was maintained overnight at 65—73°C, then poured on ice, and extracted with dichloromethane. The organic layer was washed twice with 1 N sodium hydroxide, then with water and dried over sodium sulphate. After distilling off the solvent the residue was dissolved in 4 l boiling pentane. After cooling at + 10°C a total of 878.4g crystals (m.p. 42—43°C) were obtained in two crops which were recrystallized from 5 l pentane, giving 779.1g (83.9% yield) white crystals of m.p. 43—43.5°C.

Example 2

0.325g (0.1 × $10^{-4}$ moles Pt) of a complex of chloroplatinic acid and a low molecular weight methylvinylsiloxane was dissolved in 6.6g analytical purity toluene and charged to a flask fitted with a condenser, agitator and droppng funnel. This was then heated to about 90°C and to the heated mixture was added a solution containing 37.1g of the methylhydrogensiloxane reactant of Example 1, 19.2g of salicylic acid methallyl ester and 50g of analytical purity toluene. The flask temperature was raised to reflux temperature (115°C) during addition and maintained there for 3 hours.

The solvent was then removed by vacuum distillation and the residue allowed to cool. After filtration there was obtained 53.3g of a straw-coloured, slightly hazy liquid having the following properties:

| | |
|---|---|
| Viscosity (25°C) | 53.8 mPa.s |
| λ max | 305 nm |
| Extinction coefficient (Molar) | 3475 |
| Extinction coefficient (1% in $CH_2Cl_2$) | 61.6 |

**Claims**

1. Organosilicon compounds which are

(1) silanes represented by the general formula

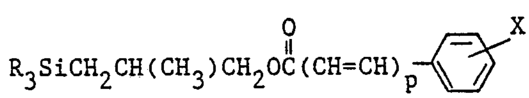

or

(2) siloxanes having at least one unit represented by the general formula

(i) 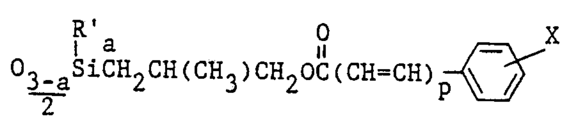

any other units present in the said siloxanes being those represented by the general formula

(ii) $$Z_bSiO_{\frac{4-b}{2}}$$

in which general formulae R represents a halogen atom, an alkyl group having from 1 to 4 carbon atoms or a phenyl group, an alkoxy group having less than 8 carbon atoms or an alkoxyalkoxy group having less than 8 carbon atoms, R′ represents an alkoxy group having less than 8 carbon atoms, an alkoxyalkoxy group having less than 8 carbon atoms, a methyl group or a phenyl group, X represents a hydroxyl group, methoxy group or ethoxy group when $p$ = 1 or a hydroxy group when $p$ = 0, Z represents a hydrogen atom, a monovalent hydrocarbon group or a monovalent halogenated hydrocarbon group, $a$ 0, 1 or 2, $b$ is 0, 1, 2 or 3 and $p$ is 0 or 1.

2. Organosilicon compounds as claimed in Claim 1 wherein $p = 1$ and X represents the methoxy group or the ethoxy group.

3. Siloxanes as claimed in Claim 1 or Claim 2 wherein at least 70 percent fo the total R′ and Z substituents are methyl groups.

4. A process for the preparation of organosilicon compounds as defined in Claim 1 which comprises reacting together (A) a compound represented by the general formula

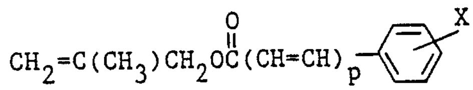

wherein $p = 0$ or 1 and X represents a hydroxyl, methoxy or ethoxy group when $p = 1$ or a hydroxyl group, when $p$ is 0 and (B) an oganosilicon compound which is a silane represented by the general formula

$$R_3SiH$$

or a siloxane having in the molecule at least one unit of the general formula

$$O_{\frac{3-a}{2}}\overset{R'_a}{\overset{|}{Si}}H$$

any other units present in the siloxane being those reprsented by the general formula

(ii) $$Z_bSiO_{\frac{4-b}{2}}$$

wherein R′, Z, $a$ and $b$ are as defined in Claim 1.

5. A process as claimed in Claim 4 wherein $p = 1$ and X represents the methoxy group or the ethoxy group.

6. A process as claimed in Claim 4 or Claim 5 wherein there is present as a catalyst for the reaction a compound or a complex of a platinum metal.

7. A sunscreen composition containing an organosilicon compound as claimed in any one of Claims 1 to 3 inclusive.

**Patentansprüche**

1. Organosiliciumverbindungen, dadurch gekennzeichnet, daß es sich hierbei um

(1) Silane der allgemeinen Formel

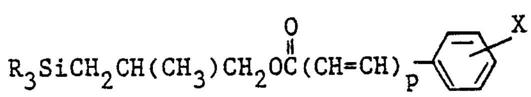

oder

(2) Siloxane handelt, die wenigstens eine Einheit der allgemeinen Formel

(i) 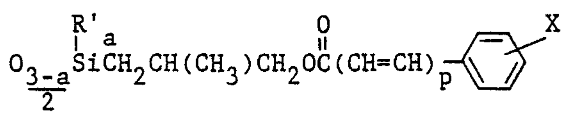

aufweisen und bei denen irgendwelche sonst vorhandene Einheiten die allgemeine Formel

(ii) $$Z_bSiO_{\frac{4-b}{2}}$$

haben, wobei in diesen allgemeinen Formeln R für ein Halogenatom, eine Alkylgruppe mit 1 bis 4 Kohlenstoffatomen oder eine Phenylgruppe, eine Alkoxygruppe mit weniger als 8 Kohlenstoffatomen oder eine Alkoxyalkoxygruppe mit weniger als 8 Kohlenstoffatomen steht, R′ eine Alkoxygruppe mit weniger als 8 Kohlenstoffatomen, eine Alkoxyalkoxygruppe mit weniger als 8 Kohlenstoffatomen, eine Methylgruppe oder eine Phenylgruppe ist, X eine Hydroxygruppe, eine Methoxygruppe oder eine Ethoxygruppe bedeutet, falls p für 1 steht, oder eine Hydroxygruppe ist, falls p für 0 steht, Z ein Wasserstoffatom, eine einwertige Kohlenwasserstoffgruppe oder eine einwertige halogenierte Kohlenwasserstoffgruppe ist, a für 0, 1 oder 2 steht, b für 0, 1, 2 oder 3 steht und p für 0 oder 1 steht.

2. Organosiliciumverbindungen nach Anspruch 1, dadurch gekennzeichnet, daß p für 1 steht und X eine Methoxygruppe oder eine Ethoxygruppe bedeutet.

3. Siloxane nach Anspruch 1 oder 2, dadurch gekennzeichnet, daß wenigstens 70 Prozent der gesamten Substituenten R′ und Z Methylgruppen sind.

4. Verfahren zur Herstellung der Organosiliciumverbindungen gemäß Anspruch 1, dadurch gekennzeichnet, daß man (A) eine Verbindung der allgemeinen Formel

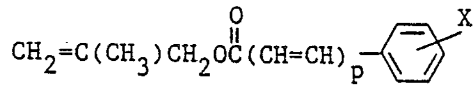

worin p für 0 oder 1 steht und X eine Hydroxygruppe, Methoxygruppe oder Ethoxygruppe ist, falls p für 1 steht, oder eine Hydroxygruppe bedeutet, falls p für 0 steht, und (B) eine Organosiliciumverbindung, welche eine Silan der allgemeinen Formel

$$R_3SiH$$

oer ein Siloxan ist, das in seinem Molekül wenigstens eine Einheit der allgemeinen Formel

$$O_{\frac{3-a}{2}}\overset{R'_a}{\overset{|}{Si}}H$$

enthält, wobei irgendwelche sonstige, in diesem Siloxan vorhandene Einheiten die allgemeine Formel

(ii) $$Z_bSiO_{\frac{4-b}{2}}$$

haben, worin R′, Z, a und b wie im Anspruch 1 definiert sind, miteinander umsetzt.

5. Verfahren nach Anspruch 4, dadurch gekennzeichnet, daß p für 1 steht und X eine Methoxygruppe oder eine Ethoxygruppe ist.

6. Verfahren nach Anspruch 4 oder 5, dadurch gekennzeichnet, daß man in Anwesenheit einer Verbindung oder eines Komplexes eines Platinmetalles als Katalysator arbeitet.

7. Sonnenschutzzusammensetzung, dadurch gekennzeichnet, daß sie eine Oranosiliciumverbindung gemäß irgendeinem der Ansprüche 1 bis 3 enthält.

**Revendications**

1. Composés organosiliciques qui sont

(1) des silanes représentés par la formule générale

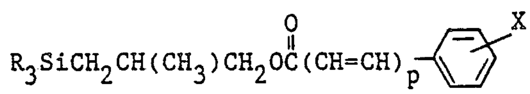

ou

(2) des siloxanes dont au moins un motif est représenté par la formule générale

(i) 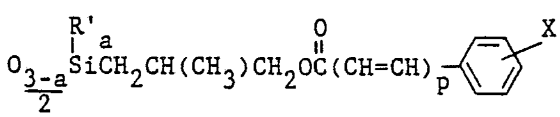

tous les autres motifs présentes dans lesdits siloxanes étant de ceux représentés par la formule générale

(ii) $$Z_bSiO_{\frac{4-b}{2}}$$

formules générales dans lesquelles R représente un atome d'halogène, un groupe alkyle ayant 1 à 4 atomes de carbone ou un groupe phényle, un groupe alcoxy ayant moins de 8 atomes de carbone ou un groupe alcoxyalcoxy ayant moins de 8 atomes de carbone, R′ représente un groupe alcoxy ayant moins de 8 atomes de carbone, un groupe alcoxyalcoxy ayant moins de 8 atomes de carbone, un groupe méthyle ou un groupe phényle, X représente un groupe hydroxyle, un groupe méthoxy ou un groupe éthoxy lorsque *p* = 1 ou un groupe hydroxyle lorsque *p* = 0, Z représente un atome d'hydrogène, un groupe hydrocarboné monovalent ou un groupe hydrocarboné halogéné monovalent, *a* est 0, 1 ou 2, *b* est 0, 1, 2 ou 3 et *p* est 0 ou 1.

2. Composés organosiliciques tels que revendiqués dans la revendication 1, dans lesquels *p* = 1 et X représente le groupe méthoxy ou le groupe éthoxy.

3. Siloxanes tels que revendiqués dans la revendication 1 ou la revendication 2, dans lesquels au moins 70 pour cent des substituants R′ et Z totaux sont des groupes méthyle.

4. Un procédé pour la préparation de composés organosiliciques tels que définis dans la revendication 1, qui consiste à faire réagir (A) un composé représenté par la formule générale

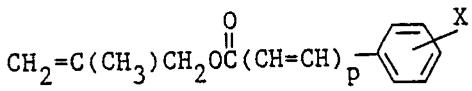

où *p* = 0 ou 1 et X représente un groupe hydroxyle, méthoxy ou éthoxy lorsque *p* = 1 ou un groupe hydroxyle lorsque *p* = 0, avec (B) un composé organosilicique qui est un silane représenté par la formule générale

$$R_3SiH$$

ou un siloxane dont la molécule contient au moins un motif de formule générale

$$\begin{array}{c} R' \\ |\ a \\ O_{\frac{3-a}{2}}SiH \end{array}$$

tous les autres motifs présents dans le siloxane étant de ceux représentés par la formule générale

(ii) $$Z_bSiO_{\frac{4-b}{2}}$$

où R′, Z, *a* et *b* sont comme définis dans la revendication 1.

5. Un procédé tel que revendiqué dans la revendication 4, dans lequel *p* = 1 et X représente le groupe méthoxy ou le groupe éthoxy.

6. Un procédé tel que revendiqué dans la revendication 4 ou la revendication 5, dans lequel un composé ou un complexe d'un métal du groupe du platine est présente à titre de catalyseur pour catalyser la réaction.

7. Une composition anti-solaire contenant un composé organosilicique tel que revendiqué dans l'une quelconque des revendications 1 à 3 inclusivement.